Europäisches Patentamt

European Patent Office

Office européen des brevets

(11) Publication number: **0 230 286 B1**

# EUROPEAN PATENT SPECIFICATION

(45) Date of publication of patent specification: **13.03.91**

(51) Int. Cl.⁵: **C07C 67/03**, C07C 67/08, C07C 67/10, B01J 21/02, B01J 21/06, B01J 23/14

(21) Application number: **87100552.6**

(22) Date of filing: **16.01.87**

(54) Method of synthesizing esters.

(30) Priority: **16.01.86 JP 5041/86**

(43) Date of publication of application:
**29.07.87 Bulletin 87/31**

(45) Publication of the grant of the patent:
**13.03.91 Bulletin 91/11**

(84) Designated Contracting States:
**CH DE FR GB IT LI SE**

(56) References cited:
**DE-C- 1 173 473**
**FR-A- 2 295 010**

(73) Proprietor: **Japan Tobacco Inc.**
**2-1 Toranomon, 2-Chome**
**Minato-Ku Tokyo 105(JP)**

(72) Inventor: **Matsushita, Hajime 301 Nihon**
**Tabako-Umegaoka**
**Daiichi-Ap. 39-9, Umegaoka**
**Midori-ku Yokohama-shi(JP)**
Inventor: **Shibagaki, Makoto 403 Nihon**
**Tabako-Dobashi**
**Daiichi-Ap. 5-6-1, Dobashi**
**Miyamae-ku Kawasaki-shi(JP)**
Inventor: **Takahashi, Kyoko**
**1-12-19, Setagaya**
**Setagaya-ku Tokyo(JP)**

(74) Representative: **Popp, Eugen, Dr. et al**
**MEISSNER, BOLTE & PARTNER Widenmayer-**
**strasse 48 Postfach 86 06 24**
**W-8000 München 86(DE)**

## Description

The present invention relates to a method of synthesizing esters, comprising a carboxylic acid with an alcohol, an ester with a carboxylic acid, or an ester with an alcohol in the presence of a catalyst in a gaseous or liquid phase.

Esters have been used as organic solvents in a variety of industrial fields. Many esters are aromatic and play an important role in perfumes. In addition, in the field of organic synthesis, ester radicals are effectively used to protect or activate carboxylic acid. For these reasons, esters have received a great deal of attention in the field of synthesis of fine chemicals.

As described above, esters constitute important compounds so that many conventional methods of synthesizing esters are known. For example, these methods are dehydration using carboxylic acid and alcohol, transesterification, o-acylation of alcohol using acylating reagents (e.g., acid halides, acid anhydrides, and ketenes) , and addition of carboxylic acid to olefins.

Among these conventional methods, the easiest, low-cost, general-purpose method is dehydration using carboxylic acid and alcohol. In order to accelerate dehydration, a catalyst is used. When acid catalysts are used, esterification by dehydration is a reversible reaction. Based on this assumption, in order to shift reaction equilibrium to esters as the objects so as to increase their yields, in general, suitable dehydration agents are used, dehydration such as azeotropic dehydration is performed, or an excessive amount of alcohol (in the case of inexpensive alcohol such as methanol and ethanol) is used.

The second popular synthesizing method of esters is transesterification. This method use inexpensive esters and synthesize them to expensive esters having high economic values. Catalysts are also used in this method.

Acid catalysts and metal alcoholates are generally used in the esterification described above.

Typical examples of acid catalysts are as follows. The first type of acid catalysts is represented by homogenous acid catalysts. The homogenous acid catalysts include mineral acids (e.g., sulfuric acid, hydrochloric acid, and phosphoric acid), organic acids (e.g., aromatic sulfonic acids), and Lewis acids (e.g., eterated boron fluoride). The second type of acid catalysts is represented by heterogeneous acid catalysts. The heterogeneous acid catalysts include solid acids or super-strong solid acids, such as acidic ion exchange resins (Chem. Ind. (London), 1967, 825), active charcoal carrying heteropoly-acid (Japanese Patent Disclosure (Kokai) No. 57-130954; and Chem. Lett. , 1981, 663), fluorinated sulfonic acid type resins (Synthesis., 1978, 929), and metal-oxides carrying sulfuric acid radicals (Japanese Patent Disclosure (Kokai) No. 57-40444).

Conventional metal alcoholate catalysts for synthesizing esters are as follows. Titanium tetraalcoholates as a homogenous catalyst of this type have been industrially used as transesterification catalyst. Examples of a heterogeneous catalyst of this type are catalysts obtained by reacting metal alcoholates with alumina, silica, silica-alumina, and the like to obtain solid catalysts (Japanese Patent Disclosure (Kokai) No. 52-75684).

The conventional methods of synthesizing esters by dehydration and transesterification, using the known catalysts, pose the following problems.

<Common Problem for Acid Catalysts>

Esterification using acid catalysts tends to cause a side reaction for producing eters upon dehydration of alcohol. When acidity is increased to enhance the catalyst activity, the side reaction tends to occur frequently. In addition to this common problem, the following problems for homogeneous or heterogeneous acid catalysts are posed.

<Homogeneous Acid Catalysts>

In a method using a homogeneous acid catalyst such as sulfuric acid, a reaction vessel is inevitably corroded. In addition, cleaning or the like is required to remove the catalyst at the end of reaction. This process necessarily accompanies the problem of effluent.

<Heterogeneous Acid Catalysts>

Use of a heterogeneous acid catalyst eliminates the problem of corrosion. In addition, the catalyst can be easily removed from the reaction system. However, the acid ion exchange resin has a low heat resistance and is subjected to insufficient activation.

The fluorinated sulfonic acid type resins obtained by improving the above drawbacks are super-strong acid ion exchange resins, having a high heat resistance and a high resistance to chemicals. However, they have short service life and are expensive.

The catalysts of active charcoal carrying heteropoly-acid are excellent solid catalysts for synthesizing ethyl acetate at a high yield when esterification is performed by a gaseous reaction between acetic acid and ethanol. However, in order to synthesize an ester having a high molecular weight, a liquid-phase reaction must be performed. In this case, elution of heteropoly acid is undesirably caused.

Metal-oxide catalysts carrying sulfuric acid radicals are super-strong solid acid catalysts and suitably used for esterification by dehydration. However, high chemical stability cannot be obtained depending on treatment methods for carrying sulfuric acid radicals on metal oxides. In addition, the resultant esters are undesirably colored.

<Metal Alcoholate Catalysts>

Metal alcoholates such as aluminum alcoholates are useful catalysts capable of causing esterification under mild conditions. In particular, even if the reaction substrate include double bonds or halogen atoms, substantially no by-products are produced, and coloring of the resultant esters can be prevented. When an ester of a high molecular weight which cannot be subjected to refinement such as distillation is to be produced, the metal alcoholate catalysts can be effectively used. Since a reaction solution becomes an alkaline solution, corrosion of the reaction vessel can be suppressed. However, most of metal alcoholates are liquids, and therefore, it is difficult to recover them after the reaction, thus posing the common problem for homogeneous catalysts. In addition, the metal alcoholates tend to react with water to easily lose activity.

The solid metal alcoholate catalysts disclosed in Japanese Patent Disclosure (Kokai) No. 52-75684, that is, the solid metal alcoholate catalysts prepared by carrying metal alcoholates in alumina, silica, silica-alumina, and the like, maintain the above-mentioned properties of metal alcoholates as well as the properties of heterogeneous catalysts. However, in order to prepare these catalysts of this type, complicated operations such as a reaction of a transition-metal alcoholate with hydroxyl radicals present on the surface of a carrier at a high temperature are required. The same complicated operations are required in other methods of preparing such catalysts.

The JP-A-1 7706/1961 discloses the use of silica gel as a catalyst in esterification of methacrylic acid with alcohols, but not as a catalyst for esterification of carbonic acids in general. The DE-A-1 173 473 refers to $TiO_2$ as a catalyst. The production of that catalyst, which is not to be partially dehydrated is highly complex and expensive, and is therefore to be disregarded for the purposes to be used here.

It is an object of the present invention to provide a method of synthesizing esters, which has the same advantages as those of esterification using heterogeneous catalysts and those of that using metal alcoholates, wherein preparation of a catalyst is easy unlike in preparation of conventional catalysts described above, and inexpensive catalysts can be used.

In order to achieve the above object of the present invention, there is provided a method of synthesizing esters, comprising reacting a carboxylic acid with an alcohol, an ester with a carboxylic acid, or an ester with an alcohol in a liquid or gaseous phase in the presence of a catalyst, wherein the catalyst comprises a partially dehydrated solid of a metal hydroxide whose metal is aluminum, tin or zirconium.

The gist of the present invention lies in the fact that a catalyst is a partially dehydrated solid of a metal hydroxide whose metal is aluminium, tin or zirconium. The partially dehydrated solid is prepared such that a corresponding metal hydroxide is heattreated under the conditions for preventing the metal hydroxide from being completely dehydrated to an oxide. In general, when zirconium hydroxide is treated at or above $500°C$ and atmospheric pressure, it is completely dehydrated to obtain zirconia ($ZrO_2$). However, if the heat treatment is performed at about $300°C$, a partially dehydrated solid can be obtained in a stable state. In other words, if the heat treatment is performed under such moderate conditions, the weight of the hydroxide is reduced by about 17% in one hour but is not reduced thereafter. The partial dehydration described above occurs in metal hydroxides other than zirconium hydroxide within the scope of this invention.

The catalyst material is a hard white solid but amorphous. For this reason, such a catalyst material cannot be analyzed by X-ray diffraction, and the detailed structure of the material is unknown. However, if the structure of the material is deduced on the fact of partial dehydration of the hydroxide, it is assumed that the material has a metal-oxygen-metal bond and hydroxyl radicals directly bonded to metal atoms are also left in the material. The catalyst material is insoluble in organic solvents (e.g., alcohol) and water and serves as a stable material as a heterogeneous catalyst. It is also found that this material has a low surface acidity and an ion exchange property for various ions.

The above catalyst materials can be easily prepared at low cost such that the metal oxides, metal chlorides, and metal salts, all of which are contained in minerals in a relatively large amount, are converted into hydroxides and the resultant hydroxides are dried and partially dehydrated. In this point, the catalyst materials of the present invention are advantageous over the conventional metal alcoholate catalysts. The catalyst material of the present invention can be ground to pieces having a predetermined size, and the pieces may be used without modifications. Alternatively, the catalyst material of the present invention may be carried on a proper carrier such as alumina, active charcoal, silica gel, silica-alumina, zeolite, or the like.

Ester synthesis according to the present invention can be performed in either a gaseous or liquid phase. In this case, excluding the fact that the above catalyst material is used, techniques in the conventional synthesizing methods of esters can be used without modifications. For example, the conventional methods and apparatuses for eliminating water or alcohol produced in the progress of reactions can be used without modifications.

If liquid-phase dehydration is performed, 0.01 to 100 mmol, preferably 0.1 to 10 mmol of an alcohol are dissolved in 1 mmol of a carboxylic acid. The resultant solution is then heated in the presence of 0.1 to 10 g and preferably 1 to 3 g of the above catalyst material. In order to the reaction is performed in a diluted solution, or in order to perform azeotropic dehydration, any other suitable solvent may be used to increase a reaction temperature. If liquid-phase transesterification is performed, 1 mmol of a carboxylic acid or alcohol is dissolved in a sufficient amount of an ester. The resultant solution is heated in the presence of 0.1 to 10 g and preferably 1 to 3 g of the catalyst material, thereby producing an ester of interest.

In any liquid-phase reaction described above, the catalyst material is recovered by filtering at the end of reaction, and the filtered solution is dried and distilled to obtain a product. The recovered catalyst can be reused without modifications.

If a gaseous-phase reaction is performed, the above catalyst material is filled in a suitable reaction tube to prepare a catalyst bed. While the tube is heated to a temperature suitable for the reaction, the source material is continuously supplied to the tube. In this case, the source material may be a mixture of an alcohol and a carboxylic acid or a mixture of an alcohol or carboxylic acid and an ester. A proper carrier gas may be used to supply the source material. A cooling device using water, ice, or any other coolant is arranged at the outlet port of the reaction tube to capture by condensation the gas flowed out from the reaction tube. The captured liquid contains a nonreacted material in addition to the product. The captured liquid may often contain a by-product. The product can be separated in the same manner as in the liquid-phase reaction described above.

If the source material is an alcohol, carboxylic acid, ester, and the like, all of which have a low boiling point, an autoclave is used to effectively synthesize a desired ester. In this case, the operations similar to the liquid-phase reaction can be performed.

According to the method of synthesizing esters of the present invention, desired esters can be produced at a high yield. In addition, since acidity of the catalyst material is low, the by-product caused by dehydration of alcohol is very small in only the high-temperature range. Therefore, coloring of the product which is caused by the conventional acid catalyst can be substantially eliminated, and high-quality esters can be produced.

The catalyst used in the present invention is substantially free from swelling or elution and has a high heat resistance and a high resistance to chemicals. Even if the catalyst is continuously used for a long period of time, activity of the catalyst is not degraded. Therefore, the catalyst can be reused and does not produce an industrial waste, thus providing industrial advantages.

Examples of preparation of catalysts used in the present invention, and examples of synthesis of esters according to the present invention will be described below.

Example 1 (Preparation of Catalyst)

After 200 g of zirconium oxychloride (octahydrate) were dissolved in 10 ℓ of deionized water, the resultant mixture was stirred and a 1N aqueous solution of sodium hydroxide was gradually added thereto to set pH = 6.80. A hydrated gel of zirconium hydroxide was precipitated. The hydrated gel was filtered and washed with deionized water. Washing was continued until no more chloride ions were detected in the filtered solution. The resultant hydrated gel was cut into pieces by a knife, and the pieces were placed on a

4

glass plate and dried at room temperature, thereby obtaining 90 g of zirconium hydroxide.

The resultant zirconium hydroxide (20 to 60 meshes) was heated at 300°C in the atmospheric pressure for 5 hours to obtain a partially dehydrated solid of zirconium hydroxide. In this case, the weight was reduced by about 17% in the heat treatment.

Example 2 (Preparation of Catalyst)

328 g of sodium aluminate were dissolved in 3 ℓ of deionized water to prepare an aqueous solution of sodium aluminate, and 336 g of sodium hydrogencarbonate were dissolved in 2 ℓ of water to prepare an aqueous solution of sodium hydrogencarbonate. The aqueous solution of sodium hydrogencarbonate was gradually added to the aqueous solution of sodium aluminate under stirring. A hydrated gel of aluminum hydroxide was prepared. The resultant hydrated gel was filtered and washed with deionized water. Washing was continued until the water used for washing had pH = 7.5. The resultant gel was cut into pieces by a knife, and each piece was cut into a 2 cm × 2 cm × 2 cm cube. These cubes were placed on a glass plate and dried at room temperature to prepare opaque aluminum hydroxide particles.

The resultant aluminum hydroxide (24 to 60 meshes) was heated at 300°C and the atmospheric pressure for 5 hours to prepare a partially dehydrated solid of aluminum hydroxide.

Example 3 (Preparation of Catalyst)

After 261 g of tin tetrachloride were dripped in 4 ℓ of deionized water, the mixture was stirred and 28% ammonia water was gradually added to the mixture under stirring to set pH = 7.0, thereby obtaining a hydrated gel of tin hydroxide. The resultant hydrated gel was filtered and washed with deionized water until no more chloride ions were detected in the filtered solution. The resultant hydrated gel was cut into pieces by a knife, and the pieces were placed on a glass plate and dried at room temperature, thereby preparing transparent tin hydroxide.

The resultant tin hydroxide (24 to 60 meshes) was heated at 300°C and the atmospheric pressure for five hours to prepare a partially dehydrated solid of tin hydroxide.

Example 4    (Ester Synthesis Using Partially Dehydrated Solid of Zirconium Hydroxide as Catalyst: Gaseous-Phase Reaction No.I)

By using 2 g of the partially dehydrated solid of zirconium hydroxide prepared in Example 1 were used as a catalyst and were fixed in a glass tube (inner and outer diameters of 4 mm and 6 mm) in a 200°C electric furnace. The glass tube was used as a reaction vessel, and the following ester synthesis was performed.

Acetic acid and ethanol were used as source materials and were mixed at a molar ratio of acetic acid to ethanol of I : 5. The mixture was supplied by a microfeeder to the glass tube at a rate of 10 mℓ/hour. In this case, a carrier gas flow was a nitrogen gas flow (I mℓ/sec). The source materials were evaporated in the glass tube and brought into contact with the catalyst. The resultant gas was then cooled with water and captured. When the captured liquid was analyzed by a gas chromatograph, 99% of acetic acid disappeared, and a peak of a corresponding amount of ethyl acetate was observed. In this case, no other by-products were found.

Subsequently, following the same procedures as described above, except that a carboxylic acid excluding acetic acid and an alcohol excluding ethanol were used as source materials and that the temperature and the amount of catalyst were changed, ester synthesis was performed.

The results in Example 4 are summarized in Table I. The conversion rate represents a ratio of the amount of a source material lost in the reaction to the total amount of source material supplied for the reaction, and the selectivity rate represents a ratio of the amount of source material converted into a desired product to the amount of source material lost in the reaction.

EP 0 230 286 B1

Table 1

| Carboxylic acid Alcohol Molar ratio | Reaction product | Temperature (°C) | Catalyst amount (g) | Conversion rate (%) | Selectivity rate (%) |
|---|---|---|---|---|---|
| $CH_3COOH$  $C_2H_5OH$<br><br>1 : 5 | $CH_3COOC_2H_5$ | 200 | 2 | *1<br>99 | 100 |
| $CH_3COOH$  ⬡—OH<br><br>1 : 5 | $CH_3COO$—⬡ | 200 | 2 | *1<br>100 | 100 |
| $C_5H_{11}COOH$  $C_3H_7OH$<br><br>1 : 130 | $C_5H_{11}COOC_3H_7$ | 215 | 2 | *1<br>100 | 87 |
| ⬡—COOH  $C_2H_5OH$<br><br>1 : 170 | ⬡—$COOC_2H_5$ | 215 | 2 | *1<br>100 | 91 |
| ⬡—COOH  $C_4H_9OH$<br><br>1 : 10 | ⬡—$COOC_4H_9$ | 250 | 2 | *1<br>100 | 78 |

6

| Carboxylic acid / Alcohol / Molar ratio | Reaction product | Temperature (°C) | Catalyst amount (g) | Conversion rate (%) | Selectivity rate (%) |
|---|---|---|---|---|---|
| $(CH_3)_3CCOOH$ : $C_2H_5OH$ = 1 : 170 | $(CH_3)_3CCOOC_2H_5$ | 250 | 2 | *1 100 | 60 |
| $CH_3COOH$ : cyclohexyl–OH = 1 : 5 | $CH_3COO$–cyclohexyl | 200 | 1 | *1 86 | 100 |
| $CH_3COOH$ : cyclohexyl–OH = 1 : 5 | $CH_3COO$–cyclohexyl | 300 | 1 | *1 100 | 96 |
| $CH_3COOH$ : cyclohexyl–OH = 1.5 : 1 | $CH_3COO$–cyclohexyl | 200 | 2 | *2 100 | 100 |
| $CH_3COOH$ : phenyl–$CH_2OH$ = 1.5 : 1 | $CH_3COOCH_2$–phenyl | 150 | 1 | *2 94 | 100 |

*1 Based on carboxylic acid    *2 Based on alcohol

Example 5    (Ester Synthesis Using Partially Dehydrated Solid of Zirconium Hydroxide as Catalyst: Gaseous-Phase Reaction No. 2)

In order to test the influences of a mixing ratio of a carboxylic acid and alcohol on the reaction, a mixing ratio of acetic acid and ethanol was changed to synthesize ethyl acetates, following the same procedures as in Example 4. The reaction temperatures and their results are shown in Table 2. It is apparent that the influences of the mixing ratio on the conversion rate and on the selectivity rate are small.

Table 2

| Molar ratio Acetic : Ethanol acid | Temperature (°C) | Conversion rate (%) | Selectivity rate (%) |
|---|---|---|---|
| 1 : 172 | 120 | 100 *1 | 100 |
| 1 : 10 | 150 | 70 *1 | 100 |
| 1 : 2 | 250 | 94 *1 | 95 |
| 1 : 1 | 250 | 73 *2 | 92 |
| 2 : 1 | 250 | 64 *2 | 100 |

*1   Based on acetic acid

*2   Based on ethanol

Example 6    (Ester Synthesis Using Partially Dehydrated Solid of Zirconium Hydroxide as Catalyst: Liquid-Phase Reaction No. I)

Two grams of the partially dehydrated solid of zirconium hydroxide prepared in Example 1 were placed as a catalyst in a 50-mℓ flask with a reflux device. In the flask, 2 mmol of acetic acid and 32 mmol of n-butyl alcohol were poured to heat and moderately reflux the mixture, thereby performing esterification. After 2 hours, a portion of the reacted solution was removed and subjected to gas chromatographic analysis. In this case, l00% of acetic acid as the source material was lost, and the peak of the corresponding amount of butyl acetate was observed. No by-products were found.

Following the same procedures as described above, ester synthesis using various carboxylic acids and alcohols was performed, and the results are summarized in Table 3.

Table 3

| Carboxylic acid (mmol) | Alcohol (mmol) | Reaction product | Reaction time (Hr) | Conversion rate (%) | Selectivity rate (%) |
|---|---|---|---|---|---|
| $CH_3COOH$ 2 | $n\text{-}C_4H_9OH$ 32 | $CH_3COOC_4H_9$ | 2 | *1 100 | 100 |
| ⬡-COOH 2 | $C_2H_5OH$ 100 | ⬡-$COOC_2H_5$ | 7 | *1 94 | 100 |
| $CH_3COOH$ 62.5 | ⬡-OH 25 | $CH_3COO$-⬡ | 6.5 | *2 68 | 100 |
| $n\text{-}C_5H_{11}COOH$ 2 | $C_2H_5OH$ 100 | $CH_3(CH_2)COOC_2H_5$ | 5.5 | *1 99.6 | 100 |

EP 0 230 286 B1

| Carboxylic acid (mmol) | Alcohol (mmol) | Reaction product | Reaction time (Hr) | Conversion rate (%) | Selectivity rate (%) |
|---|---|---|---|---|---|
| $(CH_3)_3CCOOH$ 2 | $C_2H_5OH$ 100 | $(CH_3)_3CCOOC_2H_5$ | 10 | *1 64 | 100 |
| $C_2H_5COOH$ 25 | $n-C_4H_9OH$ 62.5 | $C_2H_5COOC_4H_9$ | 8 | *1 94 | 100 |
| $CH_3COOH$ 2 | $C_2H_5OH$ 100 | $CH_3COOC_2H_5$ | 5 | *1 98 | 100 |

*1   Based on carboxylic acid          *2   Based on alcohol

EP 0 230 286 B1

Example 7 (Ester Synthesis Using Partially Dehydrated Solid of Zirconium Hydroxide as Catalyst: Liquid-Phase Reaction No. 2)

In order to test the influences of the mixing ratio of a carboxylic acid and alcohol on the liquid-phase reaction in Example 6, the mixing ratio of acetic acid and ethanol was changed to synthesize ethyl acetates, following the same procedures as in Example 6. The results are summarized in Table 4.

Table 4

| Molar ratio Acetic acid : Ethanol (mmol) (mmol) | Reaction time (Hr) | Conversion rate (%) | Selectivity rate (%) |
|---|---|---|---|
| 1 : 5 (25) (125) | 5 | 96 *1 | 100 |
| 1 : 2.5 (25) (62.5) | 5 | 86 *1 | 100 |
| 1 : 1 (25) (25) | 5 | 77 *1 | 100 |
| 2.5 : 1 (62.5) (25) | 5 | 94 *2 | 100 |

*1 Based on acetic acid

*2 Based on ethanol

Example 8 (Ester Synthesis Using Partial Dehydrated Solid of Zirconium Hydroxide as Catalyst: Liquid-Phase Reaction No. 3)

Two grams of the partially dehydrated solid of zirconium hydroxide were placed together with I mmol of acetic acid, IO mmol of benzyl alcohol, and 2 ml of toluene in a 50-ml flask having a reflux device. The mixture was heated and refluxed to esterify acetic acid and benzyl alcohol in the presence of toluene. A portion of the reacted solution was removed after one hour and was subjected to gas chromatographic analysis. IO0% of acetic acid as the source material was lost, and the peak of the corresponding amount of benzyl acetate was observed. No by-products were found.

Following the same procedures as described above, toluene was added to mixtures of other carboxylic acids and other alcohols, and the reaction was performed. The results in Table 5 were obtained.

Table 5

| Carboxylic acid (mmol) Molar Ratio | Alcohol (mmol) | Toluene (mℓ) | Reaction product | Reaction (Hr) | Conversion rate (%) | Selectivity rate (%) |
|---|---|---|---|---|---|---|
| CH$_3$OCCH (1)  1 : | ⬡-CH$_2$OH (10)  10 | 2 | ⬡-CH$_2$OCOCH$_3$ | 1 | *1 100 | 100 |
| CH$_3$COOH (62.5)  2.5 : | (structure) OH (25)  1 | 4 | (structure) OCOCH$_3$ | 5 | *2 42 | 100 |
| CH$_3$COOH (2)  1 : | C$_2$H$_5$OH (20)  10 | 4 | CH$_3$COOC$_2$H$_5$ | 5 | *1 98 | 100 |

*1  Based on carboxylic acid     *2  Based on alcohol

EP 0 230 286 B1

Example 9    (Ester Synthesis Using Partially Dehydrated Solid of Zirconium Hydroxide as Catalyst: Gaseous-Phase Transesterification)

Two grams of the partially dehydrated solid of zirconium hydroxide prepared in Example 1 were fixed as a catalyst in a glass tube (inner and outer diameters of 4 mm and 6 mm) in a 200$^\circ$C electric furnace, and ester synthesis was performed according to gaseous-phase transesterification as follows.

Ethyl acetate and n-amyl alcohol were mixed at a molar ratio of 100 : 1 (ethyl acetate : n-amyl alcohol), and the resultant mixture was supplied by a microfeeder for feeding a 1-m$\ell$/sec nitrogen gas flow to the glass tube at a rate of 10 m$\ell$/hour at room temperature. The solution was evaporated and brought into contact with the catalyst, and the resultant gas flowed out after the reaction was cooled with water. The resultant solution was captured. The captured solution was analyzed by gas chromatography. 100% of n-amyl alcohol disappeared and the peak of the corresponding amount of amyl acetate was observed. No by-products were found.

N-amyl alcohol was replaced with other alcohols, and ester synthesis was performed according to gaseous-phase transesterification.

The results are summarized in Table 6.

Table 6

| Ester | Carboxylic acid or alcohol | Reaction product | Conversion rate* (%) | Selectivity rate (%) |
|---|---|---|---|---|
| $CH_3COOC_2H_5$ | $CH_3(CH_2)_4OH$ | $CH_3COO(CH_2)_4CH_3$ | 100 | 100 |
| $CH_3COOC_2H_5$ | ⬡–COOH | $CH_3COO$–⬡ | 97 | 96 |
| $CH_3COOC_2H_5$ | ⬡–$CH_2OH$ | $CH_3COOCH_2$–⬡ | 98 | 98 |
| $CH_3COOC_2H_5$ | $CH_3(CH_2)_9OH$ | $CH_3COO(CH_2)_9CH_3$ | 99 | 99 |
| $CH_3COOC_2H_5$ | $CH_3CH_2CH(CH_2)_6CH_3$<br>$\vert$<br>OH | $CH_3CH_2CH(CH_2)_6CH_3$<br>$\vert$<br>$OCOCH_3$ | 93 | 99 |

EP 0 230 286 B1

| Ester | Carboxylic acid or alcohol | Reaction product | Conversion rate* (%) | Selectivity rate (%) |
|---|---|---|---|---|
| $CH_3COOC_2H_5$ | (menthol, —OH) | (menthyl acetate, —OCOCH$_3$) | 43 | 100 |
| $CH_3COOC_2H_5$ | $CH_3(CH_2)_4COOH$ | $CH_3(CH_2)_4COOC_2H_5$ | 27 | 84 |

\* Based on carboxylic acid or alcohol

Example 10    (Ester Synthesis Using Partial Dehydrated Solid of Zirconium Hydroxide as Catalyst:
                Liquid-Phase Transesterification)

Two grams of the partially dehydrated solid of zirconium hydroxide prepared in Example 1 were placed as a catalyst in a 50-mℓ flask with a reflux device. In the flask, 60 mmol of ethyl acetate and 2 mmol of n-amyl alcohol were poured to heat and moderately reflux the mixture, thereby performing esterification. After 8.5 hours, a portion of the reacted solution was removed and subjected to gas chromatographic analysis. In this case, 52% of n-amyl alcohol as the source material was lost, and the peak of the corresponding amount of amyl acetate was observed. No by-products were found.

Following the same procedures as described above, ester synthesis using various carboxylic acids and alcohols was performed, and the results are summarized in Table 7.

Table 7

| Ester | Alcohol | Reaction product | Reaction time (Hr) | Conversion rate* (%) | Selectivity rate (%) |
|---|---|---|---|---|---|
| $CH_3COOC_2H_5$ | $CH_3(CH_2)_4OH$ | $CH_3CCO(CH_2)_4CH_3$ | 8.5 | 52 | 100 |
| $CH_3COOC_2H_5$ | ⬡–$CH_2OH$ | $CH_3COOCH_2$–⬡ | 10 | 49 | 100 |
| $CH_3COOC_2H_5$ | $CH_3(CH_2)_9OH$ | $CH_3COO(CH_2)_9CH_3$ | 10 | 31 | 100 |
| $CH_3COOC_2H_5$ | ⬡–OH | $CH_3COO$–⬡ | 5 | 12 | 100 |

\* Based on alcohol

EP 0 230 286 B1

Example 11    (Ester Synthesis Using Partially Dehydrated Solid of Zirconium Hydroxide as Catalyst:
             Gaseous-Phase Reaction for Long Period of Time)

Following the same procedures as in Example 4 and using acetic acid and ethanol as source materials, ester synthesis was performed for a long period of time to test changes in catalyst activity.

Two grams of the partially dehydrated solid of zirconium hydroxide prepared in Example 1 were used as a catalyst and were fixed in a glass tube (inner and outer diameters of 4 mm and 6 mm) in a 200°C electric furnace. The glass tube was used as a reaction vessel, and the following ester synthesis was performed. Acetic acid and ethanol were used as source materials and were mixed at a molar ratio of acetic acid to ethanol of I : 5. The mixture was supplied by a microfeeder to the glass tube at a rate of I0 mℓ/hour. In this case, a carrier gas flow was a nitrogen gas flow (I mℓ/sec). The source materials were evaporated in the glass tube and brought into contact with the catalyst. The resultant gas was then cooled with water and captured. The captured liquid was analyzed by gas chromatography. In this case, the capture was performed once in every 2 hours. Every time the capture cycle was completed, the resultant liquid was analyzed by the gas chromatography, and the amounts of ester, and nonreacted alcohol and acid were measured.

As a result, the conversion rate based on acetic acid was 95% for I74 hours, and the selectivity ratio was kept to be I00% for I74 hours. For I74 hours, the activation ability of the catalyst was not degraded at all.

Example 12    (Ester Synthesis of Partially Dehydrated Solid of Zirconium Hydroxide as Catalyst: Liquid-
             Phase Reaction in Autoclave)

Two grams of the partially dehydrated solid of zirconium hydroxide prepared in Example 1, 10 mmol of salicylic acid, and 30 mℓ of methanol were poured in a stainless autoclave having an internal volume of I00 mℓ. The mixture was esterified at 2I0°C for 2 hours. The conversion rate of salicylic acid was I00%, and the selectivity rate thereof was 95.I%. In this case, 4.9% of phenol as a by-product was produced by decarboxylation of salicylic acid.

Following the same procedures as described above, other carboxylic acids and other alcohols were selectively used to perform the synthesis reaction. In this case, 2 g of catalyst were used, and the reaction time was 2 hours. The results are summarized in Table 8. Values in parentheses in Table 8 are amounts in mmol of carboxlic acids and alcohols.

Table 8

| Carboxylic acid | Alcohol | Reaction temperature (°C) | Conversion rate (%) | Selectivity rate (%) |
|---|---|---|---|---|
| salicylic acid (10) | methanol (700) | 210 | 100 *1 | 95.1 *3 |
| salicylic acid (10) | methanol (700) | 180 | 69 *1 | 99.6 *4 |
| acetic acid (520) | ℓ-menthol (10) | 210 | 99 *2 | 99 |
| acetic acid (520) | tert-butanol (30) | 210 | 81 *2 | 70 |
| acetic acid (30) | tert-butanol (315) | 210 | 50 *1 | 100 |
| cyclohexane-carboxylic acid (10) | cyclohexanol (288) | 210 | 100 *1 | 100 |
| pivalic acid (10) | isopropanol (390) | 210 | 54 *1 | 100 |
| acetic acid (350) | ethylene glycol (10) | 180 | 100 *2 | 100 |
| acetic acid (350) | glycerin (10) | 200 | 96 *2 | 100 |
| p-phthalic acid (10) | n-butanol (220) | 180 | 86 *1 | 48 *5 |
| phthalic anhydride (10) | n-butanol (220) | 180 | 69 *1 | 100 |
| phthalic anhydride (10) | n-octanol (127) | 200 | 99 *1 | 100 |
| succinic acid (10) | n-butanol (220) | 180 | 98 *1 | 100 |

*1  Based on carboxylic acid

*2  Based on alcohol

*3  Production of 4.9% of phenol was accompanied.

*4  Production of 0.4% of phenol was accompanied.

*5  Production of 52% of monoester was accompanied.

Example 13    (Ester Synthesis Using Partially Dehydrated Solid of Zirconium Hydroxide as Catalyst: Liquid-Phase Transesterification in Autoclave)

Two grams of the partially dehydrated solid of zirconium hydroxide, 20 mmol of ethyl acetate, and 10 mℓ of benzyl alcohol were mixed in a stainless autoclave having an internal volume of 100 mℓ, and the mixture was subjected to transesterification reaction at 130°C for 2 hours. 96% of benzyl alcohol was converted into benzyl acetate, and 98% of converted benzyl alcohol was converted into benzyl acetate.

Various alcohols or carboxylic acids were used in place of the above benzyl alcohol to perform the synthesis reactions. In this case, the amount of catalyst was 2 g, and the reaction time was 2 hours. The results are summarized in Table 9.

Table 9

| Ester | Carboxylic acid or alcohol | Reaction temperature (°C) | Conversion rate (%) | Selectivity rate (%) * |
|---|---|---|---|---|
| ethyl acetate | benzyl alcohol | 130 | 96 | 98 |
| ethyl acetate | cyclohexanol | 130 | 18 | 100 |
| ethyl acetate | 1-decanol | 150 | 98 | 100 |
| ethyl acetate | n-lactic acid | 180 | 84 | 85 |
| ethyl acetate | cyclohexane-carboxylic acid | 200 | 75 | 100 |

* Based on carboxylic acid or alcohol

Example 14    (Ester Synthesis Using Partially Dehydrated Solid of Titanium Hydroxide as Catalyst: Gaseous-Phase Reaction)

Two grams of the partially dehydrated solid of titanium hydroxide prepared in Example 2 were fixed as a catalyst in a glass tube (inner and outer diameters of 9 mm and II mm) in a 200°C electric furnace. The glass tube served as a reaction vessel. Following the same procedures as in Example 5, a mixing solution of acetic acid and ethanol (acetic acid : ethanol = I.5 : I (molar ratio)) was used as a source material, and esterification was performed in the gaseous phase. In this case, the source material was fed by a microfeeder at a rate of 5 mℓ/hour. Following the same procedures as in Example 5, the product was captured and analyzed by gas chromatography. 96.8% of acetic acid as the source material was lost, and the peak of the corresponding amount of ethyl acetate was observed. No by-products were found.

Subsequently, 20 mmol of cyclohexanecarboxylic acid were dissolved in I00 mℓ of isopropanol to prepare a mixture solution. Following the same procedures and conditions as described above, esterification was performed and isopropyl-cyclohexanecarboxylate was obtained at a yield of 95%.

Example 15    (Ester Synthesis Using Partial Dehydrated Solid of Aluminum Hydroxide as Catalyst: Gaseous-Phase Reaction)

Two grams of the partially dehydrated solid of aluminum hydroxide prepared in Example 2 were fixed as a catalyst in a glass tube (inner and outer diameters of 9 mm and 11 mm) in 200°C electric furnace. The glass tube served as a reaction vessel. Following the same procedures as in Example 4, 1 mmol of caproic acid was dissolved in 50 mℓ of ethanol, and the mixture was subjected to ester synthesis in the gaseous phase. The flow rate by the microfeeder was 5 mℓ/hour. Following the same procedures as in Example 4, the product was captured and analyzed by the gas chromatography. 96.8% of n-caproic acid was lost, and the presence of the corresponding amount of ester was observed.

Example 16    (Ester Synthesis Using Partially Dehydrated Solid of Aluminum Hydroxide as Catalyst: Liquid-Phase Transesterification)

Two grams of the partially dehydrated solid of aluminum hydroxide prepared in Example 2 was poured in a flask having an internal volume of 100 mℓ with 30 mℓ of ethyl acetate and 10 mℓ of benzyl alcohol. The mixture solution was heated and refluxed for 5 hours. A portion of the reacted solution was removed and its composition was analyzed by the gas chromatography. 84% of benzyl alcohol was lost, and the presence of the corresponding amount of benzyl acetate was observed.

Example 17    (Ester Synthesis Using Partially Dehydrated Solid of Tin Hydroxide as Catalyst: Gaseous-Phase Transesterification)

Two grams of the partially dehydrated solid of tin hydroxide prepared in Example 3 were fixed as a catalyst in a glass tube (inner and outer diameters of 6.5 mm and 8.5 mm) in a 200°C electric furnace. The glass tube served as a reaction vessel. Following the same procedures as In Example 4, a mixing solution of ethyl acetate and cyclohexanol (30 : I in molar ratio) was used as a source material to perform ester synthesis in the gaseous phase. In this case, the flow rate of nitrogen gas for feed the source material was 60 mℓ/min, and the feed rate of the microfeeder was 5 mℓ/hour. Following the same procedures as in Example 4, the product was captured and subjected to gas chromatographic analysis. 93% of cyclohexanol as the source material was lost, and 95% of the lost cyclohexanol was converted to an ester.

Using benzyl alcohol in place of cyclohexanol, same procedure for transesterification was conducted. In this case, 94% of benzyl alcohol was lost, and 92% of the lost benzyl alcohol was converted to a corresponding ester.

Example 18    (Ester Synthesis Using Partially Dehydrated Solid of Tin Hydroxide as Catalyst: Liquid-Phase Reaction)

Two grams of the partially dehydrated solid of tin hydroxide prepared in Example 3 were placed together with 2 mmol of propionic acid and 30 mℓ of n-propanol in a flask having an internal volume of I00 mℓ. The mixture was heated and refluxed. A portion of the reacted solution was removed after 5 hours and subjected to gas chromatographic analysis. 98% of propionic acid as the source material was lost, and the presence of the corresponding amount of ester was observed.

## Claims

1. A method of synthesizing esters, comprising reacting a carboxylic acid with an alcohol, an ester with a carboxylic acid, or an ester with an alcohol in the presence of a catalyst in a gaseous or liquid phase, characterized in that the catalyst comprises a partially dehydrated solid of a metal hydroxide whose metal is aluminium, tin or zirconium.

2. A method according to claim 1, characterized in that the partially dehydrated solid of the metal hydroxide is prepared by carrying a partially dehydrated solid on a carrier.

3. A method according to claim 1 or 2, characterized in that a source material is synthesized in the liquid phase, and the catalyst is filtered and recovered at the end of reaction.

4. A method according to claim 3, characterized in that a proper solvent is used in addition to the source material.

5. A method according to claim 3 or 4, characterized in that the source material is a compound having a low boiling point, and a reaction is performed in an autoclave.

6. A method according to claim 1 or 2, characterized in that the catalyst is filled in a proper reaction tube to constitute a catalyst bed, an evaporated source material is continuously supplied and brought into contact with the catalyst bed to allow reaction of the source material, and reacted geseous mixture is cooled and captured at an outlet port of the reaction tube.

## Revendications

1. Une méthode de synthèse des esters comprenant la réaction d'un acide carboxylique avec un alcool, d'un ester avec un acide carboxylique, ou d'un ester avec un alcool, en présence d'un catalyseur en phase gazeuse ou liquide, caractérisée en ce que le catalyseur comprend un solide partiellement déshydraté d'un hydroxyde métallique dont le métal est l'aluminium, l'étain ou le zirconium.

2. Une méthode selon la revendication 1, caractérisée en ce que le solide partiellement déshydraté de l'hydroxyde métallique est préparé en déposant un solide partiellement déshydraté sur un support.

3. Une méthode selon la revendication 1 ou 2, caracté-risée en ce qu'une matière de source est synthétisée en phase liquide, et le catalyseur est filtré et récupéré à la fin de la réaction.

4. Une méthode selon la revendication 3, caractérisée en ce qu'un solvant approprié est utilisé en plus de la matière de source.

5. Une méthode selon la revendication 3 ou 4, caracté-risée en ce que la matière de source est un composé ayant un bas point d'ébullition, et une réaction est conduite en autoclave.

6. Une méthode selon la revendication 1 ou 2, caracté-risée en ce que le catalyseur est rempli dans un tube de réaction approprié pour constituer un lit catalytique, une matière de source évaporée est alimentée en continu et amenée en contact avec le lit catalytique pour permettre le déroulement de la réaction de la matière de source, et le mélange gazeux ayant réagi est refroidi et capturé à l'ouverture de sortie du tube de réaction.

**Ansprüche**

1. Verfahren zur Synthese von Estern, umfassend das Umsetzen einer Carbonsäure mit einem Alkohol, eines Esters mit einer Carbonsäure oder eines Esters mit einem Alkohol in Gegenwart eines Katalysators in einer Gas- oder Flüssigphase, dadurch gekennzeichnet, daß der Katalysator einen teildehydratisierten Feststoff eines Metallhydroxids umfaßt, dessen Metall Aluminium, Zinn oder Zirkonium ist.

2. Verfahren nach Anspruch 1, dadurch gekennzeichnet, daß der teildehydratisierte Feststoff des Metallhydroxids durch Transportieren eines teildehydratisierten Feststoffs auf einem Träger erhalten wird.

3. Verfahren nach Anspruch 1 oder 2, dadurch gekennzeichnet, daß ein Ausgangsmaterial in der Flüssigphase synthetisiert wird und der Katalysator gefiltert und am Ende der Reaktion rückgewonnen wird.

4. Verfahren nach Anspruch 3, dadurch gekennzeichnet, daß zusätzlich zum Ausgangsmaterial ein geeignetes Lösungsmittel eingesetzt wird.

5. Verfahren nach Anspruch 3 oder 4, dadurch gekennzeichnet, daß das Ausgangsmaterial eine niedrigsiedende Verbindung ist und eine Reaktion in einem Autoklaven durchgeführt wird.

6. Verfahren nach Anspruch 1 oder 2, dadurch gekennzeichnet, daß der Katalysator unter Bildung eines Katalysatorbetts in ein geeignetes Reaktionsrohr gefüllt wird, ein verdampftes Ausgangsmaterial kontinuierlich zugeführt und mit dem Katalysatorbett in Kontakt gebracht wird, um die Umsetzung des Ausgangsmaterials zu ermöglichen, und umgesetztes gasförmiges Gemisch gekühlt und an einer Auslaßöffnung des Reaktionsrohrs aufgefangen wird.